# EUROPEAN PATENT SPECIFICATION

(11) **EP 2 894 474 B1**
(45) Date of publication and mention of the grant of the patent: **15.08.2018**
(21) Application number: 12884039.4
(22) Date of filing: 27.09.2012
(51) Int. Cl.: G01N 33/574

(54) **USE OF SPECIFIC BINDING MOLECULES AND OF RESPECTIVE KIT FOR DETECTING RENAL CANCER BLOOD BIOMARKERS**
VERWENDUNG SPEZIFISCHER BINDUNGSMOLEKÜLE UND VON ZUGEHÖRIGEM KIT ZUR DETEKTION VON BIOMARKERN FÜR RENALEN KREBS IN BLUT
UTILISATION DE MOLÉCULES DE LIAISON SPÉCIFIQUES ET D'UN KIT DE DÉTECTION DE BIOMARQUEURS SANGUINS DU CANCER DU REIN

(30) Priority: 07.09.2012 KR 20120099276
(43) Date of publication of application: 15.07.2015
(73) Proprietor: Genomine, Inc, Pohang-si, Gyeongsangbuk-do 790-834 (KR)
(72) Inventor: KIM, Dong Su, Daegu 706-100 (KR); MOON, Mi Hyang, Gumi-si Gyeongsangbuk-do 730-814 (KR); NA, Hyung Jin, Pohang-si Gyeongsangbuk-do 790-390 (KR); KIM, Kyung Min, Pohang-si Gyeongsangbuk-do 790-885 (KR)
(74) Representative: Betten & Resch
(86) International application number: PCT/KR2012/007846
(87) International publication number: WO 2014/038744

(56) References cited:
- EP-A1- 2 239 576
- WO-A1-2005/095455
- WO-A2-03/046581
- WO-A2-2004/046687
- WO-A2-2009/020343
- KR-A- 20030 086 345
- KR-A- 20050 086 658
- KR-A- 20090 014 979
- DONG SU KIM ET AL: "Panel of Candidate Biomarkers for Renal Cell Carcinoma", JOURNAL OF PROTEOME RESEARCH., vol. 9, no. 7, 2 July 2010 (2010-07-02), pages 3710-3719, XP055242181, US ISSN: 1535-3893, DOI: 10.1021/pr100236r
- KIM, DONG SU ET AL.: 'Panel of candidate biomarkers for renal cell carcinoma' J PROTEOME RES. vol. 9, no. 7, 02 July 2010, pages 3710 - 3719, XP055242181

## Description

### Technical Field

The present invention relates to the use of three specific binding molecules and of the respective kit for detecting renal cancer blood biomarkers.

### Background Art

Serving as a natural filter of the body, the kidneys perform the role of removing waste, excess water and salt from blood. One of the most common types of kidney cancer is renal cancer carcinoma, which originates in the renal parenchyma (a specialized tissue, composed of kidney cortex and medulla, where urine-producing cells aggregate). Typically, renal cell carcinoma occurs as single tumor in either of the kidneys, but occasionally spreads to both the kidneys.

Renal cell carcinoma (RCC) is not a single entity, but rather a collection of different types of tumours, which are classified into clear cell RCC, papillary RCC, chromophobe RCC, medullary RCC, unclassified RCC, kidney transitional cell carcinoma (TCC), and renal oncocytoma, with clear cell RCC accounting for 66∼75 % of kidney cancer cases, papillary RCC for approximately 15 %, and chromophoobe RCC for approximately 5 %.

According to the Robson staging system, renal cell carcinoma is diagnosed to be in stage I when the tumor is limited to the kidney parenchyma, in stage II upon involvement of perinephric fat, but remaining limited to Gerota's fascia, in stage III upon metastasis to perinephric vessels or lymph nodes, and in stage IV upon infiltration into other perinephric organs or distal metastasis. Herein, stage I is subdivided into stage Ia and Ib with a tumor cut off size of 4 cm. For stage III, the tumor is assigned with stage III/a when it infiltrates perinephric vessels such as the renal veins or inferior vena cava, and stage III/b when it metastasizes into perinephric lymph nodes.

Kidney cancer is diagnosed most in elderly people in their sixties to seventies, with the increasing tendency of incidence. Kidney cancer accounts for approximately 2.0 % and 1.2 % of all cancers diagnosed in men and women, ranking second and fifteenth, respectively. No symptoms can be detected until the tumor has grown to the degree of pushing out the organ. Hematuria is the most common symptom of kidney cancer, but is observed only in 60 % of the patients. Thus, there may also be no signs or symptoms in a person with kidney cancer, especially in the early stages of the disease. Under this situation, about 30 % of the diagnosed cases are in a stage of metastasis, with the expression of symptoms, such as dyspnea, cough, headache, etc., according to the metastasized sites.

Diagnosis of kidney cancer is, for the most part, conducted using X-ray, CT, ultrasonography, etc., followed by confirmation through biopsy. These methods, however, are limited in discriminating various types of kidney cancer, and require much time and labor. WO20009/020343 discloses NNMT, LCP1, secretagogin, NM23A, CapG, and C4aANA as kidney cancer biomarkers. NNMT discriminated normal individuals from RCC patients in plasma samples.

Kim et al (J Proteome Res, 2010, 9, 3710-3719) describe a triple combination of NNMT, FTL and hNSE as biomarkers for renal cell carcinoma. Disclosed in the present invention is a method useful for the early and non-invasive diagnosis of kidney cancer in which the three kidney cancer blood biomarkers NNMT (Nicotinamide N-methyltransferase), LCP1 (L-Plastin) and NM23A (Non-metastatic cells 1 protein) are used in combination to increase the diagnosis of kidney cancer.

### Disclosure

### Technical Problem

The use of a combination of three specific binding molecules of kidney cancer blood biomarkers and of the respective kit comprising them is an object of the present invention.

Other objects and concrete features of the present invention will be apparent from the following description.

### Technical Solution

The present invention is in its broadest sense defined by the appended claims. In a preparative experiment with a total of 190 blood samples obtained from 100 kidney cancer patients and 90 healthy persons, the present inventors found that NNMT, LCP1, and NM23A levels were higher in the blood of the patient group than in the healthy group, and thus can be useful as kidney cancer blood biomarkers. Also, a combination of the three kidney cancer blood biomarkers was found to diagnose kidney cancer accurately and effectively as measured by the ROC (Receiver Operating Characteristic) curve analysis. In detail, NNMT, which has the highest diagnostic ability among the three blood biomarkers (NNMT is better diagnostic for kidney cancer than LCP1 and NM23A; see the following Example Section) exhibits an accuracy (AUC) of 0.927 with a specificity of 95 % and a sensitivity of 69 % while a combination of the three blood biomarkers increased AUC to 0.9333 with a specificity of 95 % and a sensitivity of 77 % when applied to the stepwise logistic regression analysis, and further increased AUC to 0.948 with a specificity of 95 % and a sensitivity of 89 % when applied to the scoring method explained in the following Example Section.

Based on the data obtained in the preparative experiment with a total of 190 blood samples, a combination of the kidney cancer blood biomarkers were subjected to a blind test with 30 healthy blood samples and 29 patient blood samples using the scoring method. In this test, 29 of the 30 healthy persons were classified as being healthy and 28 of the 29 patients were determined as patients (that is, sensitivity 96.67 % = 29/30×100(%), specificity 96.55 % = 28/29×100(%)).

Provided on the basis of the experiment results is the use for detecting a kidney cancer blood biomarker in accordance with the present invention. In detail, the present invention relates to the use of a combination of three specific binding molecules of kidney cancer blood biomarkers in the in vitro diagnosis of kidney cancer, characterized in that the three specific binding molecules are selected from antibodies and aptamers and consist of: (i) a molecule binding specifically to a kidney cancer blood biomarker NNMT (Nicotinamide N-methyltransferase), (ii) a molecule binding specifically to a kidney cancer blood biomarker LCP1 (L-Plastin), and (iii) a molecule binding specifically to a kidney cancer blood biomarker NM23A (Non-metastatic cells 1 protein), wherein in the in vitro diagnosis of kidney cancer a scoring method is used that uses blood concentrations of the kidney cancer blood biomarkers NNMT (Nicotinamide N-methyltransferase), LCP1 (L-Platin) and NM23A (Non-metastatic cells 1 protein), detected by the three specific binding molecules, and wherein, according to the scoring method, individuals are assigned score 1 where the blood concentration of each kidney cancer blood biomarker exceeds a cut-off point (criterion), which is determined as a point where accuracy (AUC) of a receiver operating characteristic (ROC) curve of each biomarker is maximum, and score 0 where the blood concentration of each kidney cancer blood biomarker is equal to or less than the cut-off point (criterion), finally assigned a score ranging from 0 at minimum to 3 at maximum as the sum of 3 markers, with respect to a combination of the three biomarkers, wherein a cut-off value is given to score 1, and individuals are classified into kidney cancer patients where the score is higher than score 1 and into normal persons where the score is equal to or less than score 1. NNMT (nicotinamide-N-methyltransferase) is an enzyme that catalyzes the N-methylation of nicotinamide, and consists of a total of 264 amino acids, with a molecular weight of 29.6KDa. Reference may be made to GenBank Accession number: NM_006169 for its nucleotide sequence and to GenBank Accession number: NP_006160.1 for its amino acid sequence.

LCP-1(L-Plastin) is a family of actin-binding proteins. In humans, two ubiquitous plastin isofomrs (L and T) have been identified. The L isoform is expressed only in hemopoietic cell lineages. LCP-1 is composed of a total of 627 amino acids, with a molecular weight of 70.8 KDa and is disclosed in GenBank Accession number: NM_002298.4 for the nucleotide sequence and in GenBank Accession number:NP_002289.1 for the amino acid sequence.

NM23A is an isoform of the protein NM23, which is known for its reduced mRNA transcript levels in highly metastatic cells. It is composed of a total of 152 amino acids with a molecular weight of 16.9 KDa. Reference may be made to GenBank Accession number: NM_198175.1 for the nucleotide sequence and to GenBank Accession number:NP_000260.1 for the amino acid sequence.

The use of the present invention can produce quantitative analysis data about all of the kidney cancer blood biomarkers NNMT, LCP1 and NM23A that can be usefully utilized for medical experts such as physicians to determine the incidence, progression and/or cure of kidney cancer.

Configured to detect all the kidney cancer blood biomarkers NNMT, LCP1 and NM23A, the present invention can be very effectively applied to the early diagnosis of kidney cancer, with a high specificity of 96 % (115 of 120 healthy persons were determined healthy, 96 % = 115/120×100), and a sensitivity of 95 % (56 of 59 patients in stage 1 were discriminated as patients), as will be demonstrated in the following Example Section (for reference, NNMT, which is the highest diagnostic biomarker for kidney cancer, alone exhibited a specificity of 86 % and a sensitivity of 92 0). In addition, when used in combination according to the present invention, the three kidney cancer blood biomarkers can be very effectively used to diagnose clear cell RCC, which accounts for a majority of RCC cases, because of its high sensitivity of 96 % (for comparison, a specificity of 93 % was detected in NMMT, which is the highest diagnostic biomarker for kidney cancer).

As used herein, the term "processed sample of blood" refers to a plasma or serum sample.

As used herein, the term "kidney cancer blood biomarker" means NNMT, LCP1 or NM23A, which can be utilized to discriminate kidney cancer patients from healthy persons because they are present in higher levels in the blood in kidney cancer patients than in healthy persons.

The term "binding molecule specific for the kidney cancer blood biomarker," as used herein, refers to any molecule that binds specifically only to the kidney cancer blood biomarker, but not substantially to other proteins and which allows for the detection of the specific binding. The binding molecule specific for the kidney cancer blood biomarker are selected from antibodies and aptamers, with preference for antibodies. Here, the term "substantially" means that a nonspecific complex may be formed, although to a very low degree, between the binding molecule and non-targets. Such nonspecific complexes can be washed off with a wash solution before detection of the specific binding, as will be described later.

The term "antibody," as used herein, is intended to encompass all forms of a molecule capable of binding specifically to a renal cell carcinoma diagnostic marker according to the present invention. Thus, the antibody includes monoclonal antibodies, polyclonal antibodies, multispecific antibodies (which recognize two or more antigens or epitopes; e.g., bispecific antibodies), as well as fragments of an antibody molecule, recombinant antibodies and chemically modified antibodies, which retain an ability to specifically bind to any one of the renal cell carcinoma diagnostic markers of the present invention. Examples of antibody fragments include Fab, F(ab')₂, single chain Fv (scFv; consisting of a variable heavy chain and a variable light chain linked via an appropriate linker), Fv, and Fab/c (having one Fab and a complete Fc). The antibody fragments may be obtained by treating a whole antibody with a proteolytic enzyme, such as papain or pepsin. The immunoglobulin isotypes of the above antibodies are not specifically limited as long as they retain the ability to bind specifically to a kidney cancer blood biomarker according to the present invention, and may be any one of IgG, IgM, IgA, IgE and IgD.

In one exemplary embodiment of the present invention, the antibody binding specifically to the kidney cancer blood biomarker may be a polyclonal antibody or a monoclonal antibody. The preparation of monoclonal or polyclonal antibodies is known in the art. For example, a polyclonal antibody may be prepared by immunizing an animal, such as birds (e.g., chickens, etc.) or mammals (e.g., rabbits, goats, horse, sheep, rats, etc.), with a kidney cancer blood biomarker, followed by purification from the blood of the immunized animal using a method known in the art, such as ammonium sulfate fractionation, ion-exchange chromatography and affinity chromatography. A monoclonal antibody may be obtained by establishing a hybridoma cell line, which secretes a monoclonal antibody specific to a kidney cancer blood biomarker. A hybridoma cell line may be produced by immunizing an animal with a kidney cancer blood biomarker, extracting antibody-producing cells such as splenocytes, lymph node cells, etc., from the immunized animal, fusing the antibody-producing cells with a myeloma cell line to produce hybridoma cells from the fused cells, and identifying a hybridoma cell line producing a desired monoclonal antibody. The monoclonal antibody is then recovered from the hybridoma cells using a method known in the art. For more details on the production of polyclonal or monoclonal antibodies, reference may be made to various documents (Kohler and Milstein, European Journal of Immunology, 6:511-519, 1976; U. S. Patent No. 4,816,56; Clackson et al, Nature, 352:624-628, 1991; Marks et al, J. Mol. Biol., 222:58, 1-597, 1991; Harlow, E. and Lane, D., Using Antibodies: A Laboratory Manual, Cold Spring Harbor Press, New York, 1999;, Zola, H., Monoclonal Antibodies: A Manual of Techniques, CRC Press, Inc., Boca Raton, Florida, 1984; Coligan, CURRENT PROTOCOLS IN IMMUNOLOGY, Wiley/Greene, NY, 1991). The detecting step of a complex between a kidney cancer marker and an antibody may be achieved by an immunological analysis method known in the art, such as ELISA (enzyme-linked immunosorbent assay), sandwich immunoassay, fluoroimmunoassay, luminescent immunoassay, etc.

For example, an enzyme-conjugated secondary antibody may be used in ELISA. Examples of the enzyme available in ELISA include peroxidase (POD), alkaline phosphatase, β-galactosidase, horseradish peroxidase, urease, catalase, glucose oxidase, lactate dehydrogenase, and amylase. In addition, for example, the fluoroimmunoassay may be performed using an antibody conjugated with a fluorescent substance or a fluorophore, such as fluorescein isothiocyanate, tetramethylrhodamine isothiocyanate, substituted rhodamine isothiocyanate, dichlorotriazine isothiocyanate, Alexa, or AlexaFluoro. Examples of radioisotopes that can be linked to an antibody useful for the radioimmunoassay include tritium, iodine (¹³¹I, ¹²⁵I, ¹²³I, and ¹²¹I), phosphorous (³²P), sulfur (³⁵S), and metals (e.g., ⁶⁸Ga, ⁶⁷Ga, ⁶⁸Ge, ⁵⁴Mn, ⁹⁹Mo, ⁹⁹Tc, ¹³³Xe, etc.) . The luminescent immunoassay may be carried out with a luciferase system, a luminol-hydrogen peroxide-POD system, a dioxetane compound system, or the like.

More details may be obtained from documents (Enzyme Immunoassay, E. T. Maggio, ed., CRC Press, Boca Raton, Florida, 1980; Gaastra, W., Enzymelinked immunosorbent assay (ELISA), in Methods in Molecular Biology, Vol. 1, Walker, J.M. ed., Humana Press, NJ, 1984; and Ed Harlow and David Lane, Using Antibodies: A Laboratory Manual, Cold Spring Harbor Laboratory Press, 1999). Methods described may be conducted using sandwich immunoassay. In this regard, the step (a) of treating a blood sample from a subject or a processed sample thereof with binding molecules respectively specific for the kidney cancer blood biomarkers NNMT, LCP1 and NM23A to form individual complexes may comprise (a-i) immobilizing a kidney cancer blood biomarker-specific antibody as a capturing antibody onto a support, and (a-ii) treating the capturing antibody-immobilized support with a blood sample or a processed sample thereof to induce the formation of the complex between the capturing antibody and the kidney cancer blood biomarker, and the step (b) of detecting the complex may comprise (b-i) applying to the support a detection antibody conjugated with a label or enzyme that mediates or provides (that is, allows for) signal measurement, and (b-ii) measuring the signal mediated or provided by the label or enzyme.

Examples of the support include, but are not limited to, microspheres (resin beads, magnetic beads, etc.), particles (metal fine particles, gold colloids, etc.), and plates (microtiter plates, glass plates, silicon plates, resin plates, electrode plates, membranes, etc.). The support may be made of (i) an inorganic material, such as glass, quartz glass, alumina, sapphire, forsterite, silica, etc., or (ii) an organic material such as polyethylene, polyvinyl acetal, an acrylic resin, polycarbonate, a phenol resin, an urea resin, en epoxy resin, a melamine resin, a silicon resin, polyphenylene oxide, polysulfone, polyethylene glycol, agarose, acrylamide, nitrocellulose, nylon, latex, etc.

Immobilization of the capturing antibody onto the support may be achieved directly by adsorption (e.g., coating) or indirectly through a linker that binds to both the protein and the support.

The adsorption by which the capturing antibody is immobilized onto the support may be carried out by diluting the capturing antibody in a 0.06 M carbonate or bicarbonate buffer, pH 9.5, and bringing the dilution into contact with the support at a certain temperature for a predetermined period of time. The time and the temperature necessary for the adsorption are not imparted with particular limitations so long as those allows for sufficient adsorption. At 4°C, for example, adsorption may be performed for 72 hrs. Alternatively, 2 hrs of adsorption may be given at 37°C. After adsorption, the support may be rinsed with distilled water or ethanol and further coated with a blocking agent such as bovine serum albumin (BSA) in a buffer, e.g., PBS. When a blocking agent is applied thereto, the support may be washed with distilled water or a buffer containing no blocking agents.

When treated with a blood sample or a processed sample thereof, the capturing antibody adsorbed onto the support forms a complex with a kidney cancer blood biomarker contained in the sample. Following the formation of the complex, it may be preferable that the support be rinsed with a wash buffer such as Tween 20, or distilled water so as to remove an antibody that would be bound non-specifically, or a contaminant.

The label may be a chemical such as biotin, a fluorescent such as fluorescein isothianate, tetramethyl rhodamine isothiocyanate, etc., or a radioisotope such as iodine (¹³¹I, ¹²⁵I, ¹²³I, ¹²¹I), phosphorus (³²P), sulfur (³⁵S), etc. The enzyme may catalyze a reaction responsible for color development, fluorescence, or luminescence, like peroxidase (POD), alkaline phosphatase, β-galactosidase, horseradish peroxidase, urease, catalase, glucose oxidase, lactate dehydrogenase, amylase, luciferase, etc. For more details, reference may be made to Ed Harlow and David Lane, Using Antibodies: A Laboratory Manual, Cold Spring Harbor Laboratory Press, 1999.

Preferably, the label or the enzyme may be covalently bonded to the antibody.

Available as the detection antibody are those that recognize the Fc region of the antibody (capturing antibody). These detection antibodies may be obtained by immunizing an animal, such as a bird, a mammal, etc, with an Fc region, followed by separation and purification from the blood of the animal.

The signal measurement may be obtained using a method known in the art. When biotin is used as a label, for example, avidin or streptavidin may be conjugated to measure signals. For an enzyme, a substrate thereto may be adopted for signal measurement. By way of example, luciferin may be used for luciferase.

After inducing the formation of a complex between the kidney cancer blood biomarker and the capturing antibody (a-ii) and/or applying a detection antibody to the support, the method described may preferably further comprise washing the support with a wash buffer such as Tween 20 or distilled water to remove non-specifically bound antibodies or contaminants, as mentioned above. The method described may be performed using ELISA. In this regard, the step (a) of treating a blood sample from a subject or a processed sample thereof with binding molecules to form individual complexes may comprise (a-i) immobilizing the blood sample or the process sample onto a support, and (a-ii) treating the sample-immobilized support with a primary antibody to the kidney cancer blood biomarker, and the step (b) of detecting the complex may comprise (b-i) applying to the support an enzyme-conjugated secondary antibody, and (b-ii) measuring the activity of the enzyme.

For the immobilization of samples onto the support (by, for example, coating), the description given above may be applied.

As the enzyme, it may catalyze a reaction responsible for color development, fluorescence, or luminescence, as described above.

In the same manner as described for the detection antibody, the secondary antibody may be obtained by immunizing a mammal with the primary antibody as an antigen, followed by separation and purification from the blood of the animal.

The step of measuring the activity of the enzyme may be accomplished by adding a substrate to the enzyme and analyzing the enzymatic reaction.

According to another exemplary embodiment of the present invention, the molecule binding specifically to the kidney cancer blood biomarker may be an aptamer binding specifically to the kidney cancer blood biomarker. The aptamer may be an oligonucleotide or peptide molecule. For general details of antamers, reference may be made to documents (Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24):142727(1998)).

Also, contemplated in accordance with another aspect of the present invention is the use of a kit for detecting a kidney cancer blood biomarker. The present invention refers to the use of a kit comprising a combination of three specific binding molecules in the in vitro diagnosis of kidney cancer, characterized in that the three specific binding molecules are selected from antibodies and aptamers and consist of: (i) a molecule binding specifically to a kidney cancer blood biomarker NNMT (Nicotinamide N-methyltransferase), (ii) a molecule binding specifically to a kidney cancer blood biomarker LCP1 (L-Plastin), and (iii) a molecule binding specifically to a kidney cancer blood biomarker NM23A (Non-metastatic cells 1 protein), wherein in the in vitro diagnosis of kidney cancer a scoring method is used that uses blood concentrations of the kidney cancer blood biomarkers NNMT (Nicotinamide N-methyltransferase), LCP1 (L-Platin) and NM23A (Non-metastatic cells 1 protein), detected by the three specific binding molecules, and wherein, according to the scoring method, individuals are assigned score 1 where the blood concentration of each kidney cancer blood biomarker exceeds a cut-off point (criterion), which is determined as a point where accuracy (AUC) of a receiver operating characteristic (ROC) curve of each biomarker is maximum, and score 0 where the blood concentration of each kidney cancer blood biomarker is equal to or less than the cut-off point (criterion), finally assigned a score ranging from 0 at minimum to 3 at maximum as the sum of 3 markers, with respect to a combination of the three biomarkers, wherein a cut-off value is given to score 1, and individuals are classified into kidney cancer patients where the score is higher than score 1 and into normal persons where the score is equal to or less than score 1. In the kit, the molecules specific for the kidney cancer blood biomarkers are antibodies or aptamers, the former being preferred.

In the kit, the molecules specific for the kidney cancer blood biomarkers may be in a form immobilized onto a support or as isolates. When the antibodies take isolate forms, the kit may further include a support. In this context, the support may be a microsphere, a particle, or a plate, with a preference for a microsphere. More preferable is a resin bead.

In an exemplary embodiment, the kit may further include a secondary antibody or a detection antibody in a form conjugated with a label or enzyme allowing for signal measurement, or as an isolate. When the secondary antibody or the detection antibody exists as an isolate, the kit may further include the label or enzyme. The kit may further include a wash buffer, a buffer for diluting the samples or the antibodies, an enzyme substrate, a reaction stopper, etc.

The use of blood levels of the kidney cancer blood biomarkers NNMT, LCP1 and NM23A in diagnosing kidney cancer, may be accomplished in various manners. For instance, when respective blood levels of NNMT, LCP1, and NM23A are compared with cut-off values calculated by logistic regression analysis, the subject is diagnosed with kidney cancer if the blood levels are greater than the cut-off values, and classified as a healthy person if the blood levels are the same as or less than the cut-off values. Alternatively, results obtained according to the scoring method described in the following Example Section may be compared with cut-off values. The subject may be diagnosed with kidney cancer if the results are greater than the cut-off values, or as being healthy if otherwise. In this context, the cut-off values are criteria for classifying the subject as a patient or a normal person, and may be arbitrarily determined in consideration of diagnostic factors including specificity, sensitivity, etc. For instance, the cut-off may be determined as a value at which a specificity of 95 % is obtained as measured by ROC curve analysis or as a value at which a maximum AUC is obtained on an ROC curve (that is, a value at which an area established by two imaginary vertical and horizontal lines passing a point on an ROC curve is maximized). The disclosure addresses a method for detecting LCP1 serving as a kidney cancer blood biomarker, the method comprising (a) treating a blood sample from a subject or a processed sample thereof with a binding molecule specific for the kidney cancer blood biomarker LCP1 to form a complex between the kidney cancer blood biomarker LCP1 and the binding molecule, and (b) detecting the complex.

In accordance with another aspect thereof, the disclosure addresses a method for detecting NM23A serving as a kidney cancer blood biomarker, the method comprising (a) treating a blood sample from a subject or a processed sample thereof with a binding molecule specific for the kidney cancer blood biomarker NM23A to form a complex between the kidney cancer blood biomarker NM23A and the binding molecule, and (b) detecting the complex.

LCP1 and NM23A have been known as kidney cancer biomarkers from tissue samples (Korean Unexamined Patent Application Publication No. 10-2009-0014979), but not from blood until the present invention.

To details of the detecting method of the kidney cancer blood biomarker LCP1 or NM23A, the foregoing description may be applied as it is.

### Advantageous Effects

The present invention can produce quantitative analysis data about all of the kidney cancer blood biomarkers NNMT, LCP1, and NM23A that can be usefully utilized by medical experts such as physicians to determine the incidence, progression and/or cure of kidney cancer. Particularly, marker combination and kit can be very effectively used for the early diagnosis of kidney cancer and clear cell RCC, which accounts for a majority of RCC cases.

### Description of Drawings

FIG. 1 shows median blood levels of the kidney cancer blood biomarkers NNMT, LCP1, and NM23A of a normal group in comparison with a kidney cancer group.
FIG. 2 shows ROC curves for the kidney cancer blood biomarkers NNMT, LCP1, and NM23A, individual and in combination, compared with stepwise logistic regression or scoring method.

### Best Mode

A better understanding of the present invention may be obtained through the following examples that are set forth to illustrate, but are not to be construed as limiting the present invention

### EXAMPLE: Analysis of Combined Markers for Diagnostic Performance of Kidney Cancer

### <1> Experiment process

### <1-1> Collection and treatment of blood sample

From subjects who had provided written consent, all blood samples were taken according to the procedure approved by the Institutional Review Board (IRB) of Yonsei University College of Medicine. Samples were obtained from 129 persons who were determined to have kidney cancer as measured by radiological and cytopathological diagnosis. The kidney cancer patients had an average age of 55.9 years and could be classified, according to types of kidney cancer, into: a clear cell renal cell carcinoma (clear cell RCC) group of 89 patients; papillary RCC of 6 patients; chromophobe RCC of 7 patients; kidney transitional cell carcinoma (TCC) of 8 patients; Ewing sarcoma of 1 patient; and unclassified RCC of 3 patients. The remainders were accounted for by 6 patients with benign tumor of non-renal oncocytoma or with kidney cancer-negative diagnosis, and 9 patients with benign tumors. According to the progression of kidney cancer, the 129 patients could be divided into: 59 in stage Ia/Ib; 13 in stage II; 29 in stage IIIa/IIIb; and 2 in stage IV; the remainder was not classified.

Normal samples were obtained from 120 persons who had visited the diagnostic department of Yonsei University College of Medicine, and they had a mean age of 25 years. Plasma samples were collected over 15 months. Early samples collected from 100 kidney cancer patients and 90 normal persons were used in a preparative experiment (training group sample study) for analyzing the diagnostic performance of the markers. Samples taken later from 28 patients and 30 normal persons were used in a validity test for the diagnostic peformance of the marker obtained in the preparative experiment.

General information on the samples is summarized in Table 1, below.

**TABLE 1**

| | | | | Training Group (n=190 | | | | | Test Group(n=S9) | | | | |
|---|---|---|---|---|---|---|---|---|---|---|---|---|---|
| Healthy(n=120) | | | | 90 | | | | | 30 | | | | |
| Sex | Male | Female | Age Median | | | | | | | | | | |
| | 120 | 0 | 25 | | | | | | | | | | |
| | | | | | | | | | | | | | |

| Kidney Cell Tumor(n=129) | | | | 100 | | | | | 29 | | | | |
|---|---|---|---|---|---|---|---|---|---|---|---|---|---|
| Sex | Male | Female | Age Median | | | | | | | | | | |
| | 78 | 51 | 55.9 | | | | | | | | | | |
| | | | | | | | | | | | | | |

| | Cell Type | | | Cell type | | pT stage | | | Cell type | | pT stage | | |
|---|---|---|---|---|---|---|---|---|---|---|---|---|---|
| | | | | | Ia/Ib | II | IIIa/IIIb | IV | No. | Ia/Ib | Iia/Iib | IIIa/IIIb | IV |
| | Clear Cell RCC | | | 66 | 39 | 9 | 18 | | 23 | 12 | 1 | 9 | 1 |
| | Papillary RCC | | | 6 | 3 | 1 | 1 | 1 | 0 | | | | |
| | Chromophobe RCC | | | 6 | 4 | 1 | 1 | | 1 | 1 | | | |
| | TCC | | | 7 | | | | | 1 | | | | |
| | Unclassified RCC | | | 1 | | 1 | | | 2 | | | | |
| | Ewing Sarcoma | | | 1 | | | | | | | | | |
| | Renal Oncocytoma | | | 4 | | | | | 2 | | | | |
| | Benign | | | 8 | | | | | 1 | | | | |

| | | | | | | | | | | | | | |
|---|---|---|---|---|---|---|---|---|---|---|---|---|---|
| TCC Transitional Cell Carcinoma of the Kidney | | | | | | | | | | | | | |

A blood sample was collected using a 5 mL sterile vacuum blood collection tube containing 4 mg of K₂EDTA. After being mixed with the anticoagulant, the blood sample was centrifuged for 10 min at 2,500 rpm to separate plasma as a supernatant, which was then stored at -80°C until experiment.

### <1-2> Conjugation of antibody to beads

Experiments for the preparation of recombinant proteins serving as a calibrator, and antibodies and the biotinylation of a detection antibody were carried out as described previously [Journal of Proteome Research 2010, 9:3710-3719]. The recombinant proteins were prepared from full-length cDNAs for NNMT (GenBank accession number: NM_006169), LCP1 (GenBank accession number: NM_002298.4), and NM23A (GenBank accession number: NM_198175.1).

Conjugation of an antibody to beads (Luminex Corp.) was achieved as follows. First, anti-NNMT IgG was conjugated to bead No. 63; anti-LCP1 IgG to bead No. 17; and anti-NM23A IgG to bead No. 33. Conjugation between an antibody and carboxy-coated beads was made according to the protocol of the manufacturer (Luminex Corp.). Briefly, 1×10⁶ beads were washed twice with deionized water by centrifugation, and suspended in 8 µl of sodium phosphate buffer (pH 6.2) by vortexing and sonication. To the suspension, 4 µl of N-hydroxysulfosuccinimide (sulfo-NHS; Thermo Scientific) and 4 µl of 1-ethyl-3(3-dimethylaminopropyl)-carbodiimide hydrochloride (EDC; Sigma) were added, and vortex. After being left for 20 min at room temperature, the beads were washed twice with 50 µl of a coupling solution (50 mM MES), and then the resulting bead pellet was resuspended in 20 µl of the same solution. To this suspension was added diluted 10 µg of an antibody solution (1 mg/ml), and the volume was adjusted to a total of 100 µl with the coupling solution. Incubation at room temperature for 2 hrs with rotation allowed for coupling the antibody with the beads, followed by washing twice with 200 µl of a storage/coupling solution (PBS containing 0.1 % BSA, 0.05 % Tween 20, 0.05 % sodium azide) . Resuspended antibody-conjugated beads in 100 µl of the storage solution were counted using a hematocytometer, and stored at 2∼8°C in a dark room.

### <1-3> Multiplexed microsphere immunoassay

All assays were performed by bead-based sandwich immunoassays using bead-conjugated capture antibodies, biotin-labeled detection antibodies, and phycoerythrin-labeled streptavidin (Invitrogen). Assay was done in a filterplate (Millipore Corporation) prewetted with an assay buffer [100 mM Tris HCl pH 7.4, 150 mM NaCl, 1 % BSA, 2 mM EDTA, 2 % PEG 4000, 1.2 % Synperonic F68, 0.1 % sodium azide, and 0.2 mg/mL rabbit IgG)] at room temperature. All incubations in the assay were carried out by shaking at 400 rpm. For solution drain and washing, a vacuum manifold (Millipore) was employed. Recombinant protein calibrators were used for quantitation. Bovine plasma was used as a dilution matrix of calibrator as a mimic of human plasma samples.

Before multiplexed assay setup, each single marker assay was evaluated separately and pairwise addition of bead-conjugated capture antibody was conducted. Consistency or changes of a calibration curve in the subsequent addition of a capture antibody was observed. If there was any change in the calibration curve, it was adjusted and optimized by incorporating a set of blocking conditions against nonspecific binding of individual antibody pairs. Twenty microliters of an assay buffer containing 1,000 capture antibody-conjugated beads was transferred, together with 20 µL of plasma samples or calibrators, to the filterplate. After 30 min of incubation, 10 µL of biotin-labeled detection antibody (160 µg/mL) diluted with an assay buffer was added and incubated for an additional 30 minutes. After three rounds of washing each with 100 mL of a washing solution (50 mM Tris HCl pH 7.4, 150 mM NaCl, 1 % BSA, 0.1 % sodium azide, 0.05 % Tween 20), 50 mL of phycoerythrin-labeled streptavidin (PE,4 mg/mL) in a PE solution was added and incubated for 30 minutes. Without wash, 50 µL of a PE solution was added and beads were mixed thoroughly before reading on Luminex¹⁰⁰ system (Luminex Corp.). The data were processed and analyzed using using MasterPlex CT software (Ver. 1.0; MiraiBio, Inc.) with linear regression curves.

### <1-4> Data analysis

Statistical analyses were carried out using MedCalc software (Ver.9.6.4.0; http://www.medcalc.be). Linear regression was used to generate a calibration curve, and Pearson correlation coefficient (R²) was used to assess the linearity of curves. A Wilcoxon paired sample test was used to assess the significance of difference in plasma marker concentration between the control group and the kidney cancer group (p<0.0001). Logistic regression analysis with 3 markers was used to differentiate predicted probability on the presence of tumor. Stepwise logistic regression with the 3 tumor markers of 3-plex panel was conducted and the predicted probability was used to compare with the diagnostic performance of each marker. Receiver operating characteristic (ROC) curve analysis was used to generate the value of specificity, sensitivity, and accuracy (AUC). A scoring system was also applied to facilitate the diagnostic prediction of 3-panel kidney tumor markers. Scoring was carried out as reported previously [Proc. Natl.Acad. Sci. U.S.A. 2005, 102, 7677-682], with the exception for a procedure for best cut-point (criterion) determination of each marker. The best cut-points were selected at the point of maximum AUC of each marker from the ROC analysis. On the basis of the plasma concentration of markers, individuals were assigned score 0 (≤ cut-point) or 1 (>cut-point) for each marker and finally assigned a score ranging from 0 to 3 as the sum of 3 markers.

### Mode for Invention

### <2> Experiment Results

### <2-1> Analysis of diagnostic performance of individual markers

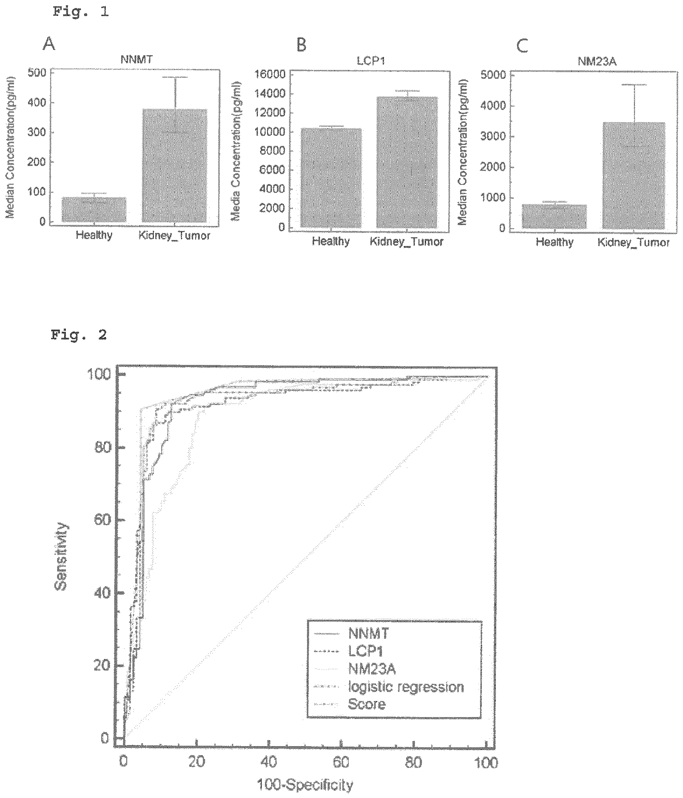

Plasma concentrations of the individual markers were measured in a total of 190 plasma samples (90 normal individuals and 100 patients with kidney cancer) and the mean values are depicted in FIG. 1. As shown in FIG. 1, the median NNMT concentration in patients with kidney cancer was 420 pg/mL, which is approximately 6.2-fold higher than 68 pg/mL for normal individuals. The median concentration of LCP1 in control individuals and patients with kidney cancers was 10,385 and 13,789 pg/mL, respectively, with an approximately 1.3-fold difference therebetween. For NM23A, a median concentration of 780 pg/ml and 3,442 pg/ml in control individuals and patients with kidney cancer were measured, respectively, with an approximately 4.4-fold difference therebetween.

At 95 % specificity defined, the sensitivity was 69 % for NNMT, 39 % for LCP1, and 48 % for NM23A, as measured by ROC curve analysis.

### <2-2> Analysis for diagnostic performance of combined markers

In the preparative experiment with a total of 190 samples (90 normal samples and 100 patients with kidney cancer), stepwise logistic regression analysis was conducted to evaluate the diagnostic performance of the combined markers. The predictive probability was generated and used to estimate the diagnostic value by ROC analysis. The diagnostic accuracy (AUC) of logistic regression with the combined markers was 0.933 and the sensitivity at fixed specificity of 95 % was 71.6 %. Thus, when LCP1 and NM23A were combined with NNMT, both sensitivity and accuracy were improved at a specificity of 95 %, compared with NNMT alone or the other two markers used individually. As a result of the preparative experiment with 190 samples, the diagnostic accuracy of NNMT, which exhibited the highest diagnostic performance among the three individual markers, was measured to be 0.927, with a sensitivity of as low as 69 % at a fixed specificity of 95 %.

To facilitate the use of the combined markers, a scoring method was applied in the preparative experiment with a total of 190 samples (90 normal individuals and 100 patients with kidney cancer) to generate predictive diagnostic values. A cut-point (criterion) for each of the three markers was selected at an ROC curve point at a maximum AUC was obtained. The cut-point was determined to be 148 pg/ml for of NNMT, 12974 pg/ml for LCP1, and 1230 pg/ml for NM23A. When the concentration of each of the three markers was above the cut-point, a score of 1 was assigned; otherwise, a score of 0 was assigned to the subjects, with a finally assigned score ranging from 0 to 3 as the sum of 3 markers. The cut-off value as a criterion for kidney cancer was 1.

For the three combined markers, the diagnostic accuracy of the score was measured to be 0.948 (AUC), with a sensitivity of 89 % at a fixed specificity of 95 %.

Blinded test group samples including 30 normal samples and 29 patient samples were applied to validate the clinical performance of the three combined markers with the scoring method based on the cut-point determined previously. In this blinded test group analysis, 29 of the 30 normal samples and 28 of the 29 patient samples were classified correctly (sensitivity 96.67 % = 29/30×100 (%), specificity 96.55 % = 28/29x100 (%)).

For a total of 249 plasma samples used in the preparative experiment and the blinded test, diagnostic performance of each and a combination of the three markers is summarized in Table 2, below, and ROC curves for individual markers are depicted in FIG. 2.

**TABLE 2**

| | | | | | | | Specificity=95(%) | Specificity =90(%) |
|---|---|---|---|---|---|---|---|---|
| Markers | Criterion | AUC | Sensitivity (%) | Specificity (%) | +PV (%) | -PV (%) | Sensitivity(95% CI) | Sensitivity(95% CI) |
| NNMT | >147 | 0.928 | 92.3 | 875 | 88.8 | 91.3 | 71 3(12.4 - 80.6) | 80.6(65.9-95.3) |
| LCP1 | >12974 | 0.916 | 86.1 | 92.5 | 925 | 86.0 | 54.3(10.1 -88.4) | 86.8(55.7-92.3) |
| NM23 | >1230 | 0.887 | 89.9 | 80.0 | 82.9 | 88.1 | 32.(17.8 - 61.2) | 65.1(37.2-76.7) |
| NNMT,LCP1,NM23A logistic regression | >0.4436 | 0.933 | 90.7 | 91.7 | 92.1 | 90.2 | 80.6(9.3 - 91.4) | 91.5(79.1-66.1) |
| NNMT,LCP1,NM23A Score | >1 | 0951 | 90.7 | 95.8 | 95.9 | 90.6 | 90.9(45.9 -96.9) | 92.4(87.5-96.0) |

| | | | | | | | | |
|---|---|---|---|---|---|---|---|---|
| AUC: area under curve, +PV : positive predictive value, -PV : negative predictive value | | | | | | | | |

As is understood from the data of Table 2 and FIG. 2, the combined markers were improved in both diagnostic accuracy (AUC) and sensitivity at 95 % specificity, compared to individual markers.

### <2-3> Evaluation for diagnostic performance according to types and pathologic stages of kidney cancer

For a total of 249 plasma samples, diagnostic performance of NNMT and the combined markers was evaluated according to types and pathologic stages of kidney cancer, and the results are given in Tables 3 and 4, respectively.

**TABLE 3**

| | Sensitivity % | | | | | | |
|---|---|---|---|---|---|---|---|
| | Cell lype | | | | | | |
| | Clear cell carcinoma | Papillary carcinoma | Chromophobe carcinoma | TCC | Unclassified carcinoma | Benign | Oncocytoma |
| NNMT at cut-off > 147pg/ml | 93(83/89) | 100(6/6) | 86(6/7) | 100(8/8) | 100(3/3) | 100(9/9) | 50(3/6) |
| NNMT,LCP1,NM23A Score at cut-off> 1 | 96(85/89) | 100(6/6) | 86(6/7) | 100(8/8) | 100(3/3) | 56(5/9) | 33(2/6) |

**TABLE 4**

| | Specificity % | Sensitivity % | | | |
|---|---|---|---|---|---|
| | Healthy | Pathological Tumor Stage | | | |
| | | Stage Ia/Ib | Stage II | Stage IIIa/IIIb | Stage VI |
| NNMT at cut-off > 147pg/ml | 86(15/120) | 92(54/59) | 100(13/13) | 100(29/29) | 100(2/2) |
| NNMT,LCP1,NM23A Score at cut-off> 1 | 96(5/120) | 95(56/59) | 92(12/13) | 97(28/29) | 100(2/2) |

For clear cell RCC, which accounts for diagnosed RCC cases, as can be seen in Table 3, the combined markers exhibited a sensitivity of 96 %, compared to 93 % sensitivity with NNMT alone. In Table 4, sensitivity for the early stage (stage I) is observed to measure 92 % with NNMT and 95 % with the combined markers.

Taken together, the data obtained above demonstrate that a combination of NNMT, LCP1 and NM23A is very effective for the early diagnosis of kidney cancer, particularly, clear cell RCC, which accounts for the majority of kidney cancer.

## Claims

1. Use of a combination of three specific binding molecules of kidney cancer blood biomarkers in the *in vitro* diagnosis of kidney cancer, **characterized in that**
the three specific binding molecules are selected from antibodies and aptamers and consist of: (i) a molecule binding specifically to a kidney cancer blood biomarker NNMT (Nicotinamide N-methyltransferase), (ii) a molecule binding specifically to a kidney cancer blood biomarker LCP1 (L-Plastin), and (iii) a molecule binding specifically to a kidney cancer blood biomarker NM23A (Non-metastatic cells 1 protein),
wherein in the *in vitro* diagnosis of kidney cancer a scoring method is used that uses blood concentrations of the kidney cancer blood biomarkers NNMT (Nicotinamide N-methyltransferase), LCP1 (L-Platin) and NM23A (Non-metastatic cells 1 protein), detected by the three specific binding molecules, and
wherein, according to the scoring method, individuals are assigned score 1 where the blood concentration of each kidney cancer blood biomarker exceeds a cut-off point (criterion), which is determined as a point where accuracy (AUC) of a receiver operating characteristic (ROC) curve of each biomarker is maximum, and score 0 where the blood concentration of each kidney cancer blood biomarker is equal to or less than the cut-off point (criterion), finally assigned a score ranging from 0 at minimum to 3 at maximum as the sum of 3 markers, with respect to a combination of the three biomarkers, wherein a cut-off value is given to score 1, and individuals are classified into kidney cancer patients where the score is higher than score 1 and into normal persons where the score is equal to or less than score 1.

2. The use of a combination of three specific binding molecules of kidney cancer blood biomarkers according to claim 1, wherein (a) a blood sample from a subject or a processed sample thereof is treated with the combination of three specific binding molecules to form individual complexes between the kidney cancer blood biomarkers NNMT, LCP1, and NM23A and the binding molecules respectively specific thereto, and wherein (b) the complexes are detected.

3. The use of a combination of three specific binding molecules of kidney cancer blood biomarkers according to claim 1 or 2, wherein the processed sample is a plasma or serum sample.

4. The use of a combination of three specific binding molecules of kidney cancer blood biomarkers according to claim 1 or 2, wherein the binding molecules are antibodies specific for the kidney cancer blood biomarkers, respectively.

5. The use of a combination of three specific binding molecules of kidney cancer blood biomarkers according to claim 4, wherein the step (a) of treating a blood sample from a subject or a processed sample thereof with a combination of three specific binding molecules comprises:
(a-i) immobilizing the kidney cancer blood biomarker-specific antibodies as capturing antibodies onto a support; and
(a-ii) treating the capturing antibody-immobilized support with a blood sample or a processed sample thereof to induce the formation of the complexes between the capturing antibodies and the kidney cancer blood biomarkers, and
wherein the step (b) of detecting the complexes comprises:
(b-i) applying to the support a detection antibody conjugated with a label or enzyme that mediates or provides signal measurement; and
(b-ii) measuring the signal mediated or provided by the label or enzyme.

6. The use of a combination of three specific binding molecules of kidney cancer blood biomarkers according to claim 5, wherein the support is a microsphere, the microsphere being a resin bead.

7. The use of a combination of three specific binding molecules of kidney cancer blood biomarkers according to claim 5, wherein the label that mediates or provides signal measurement is biotin.

8. The use of a combination of three specific binding molecules of kidney cancer blood biomarkers according to claim 4, wherein the step (a) of treating a blood sample from a subject or a processed sample thereof with a combination of three specific binding molecules comprises:
(a-i) immobilizing the blood sample or the process sample onto a support; and
(a-ii) treating the sample-immobilized support with the primary antibodies to the kidney cancer blood biomarkers; and
wherein the step (b) of detecting the complex comprises:
(b-i) applying to the support an enzyme-conjugated secondary antibody; and
(b-ii) measuring the enzyme for activity.

9. Use of a kit comprising a combination of three specific binding molecules in the *in vitro* diagnosis of kidney cancer, **characterized in that**
the three specific binding molecules are selected from antibodies and aptamers and consist of: (i) a molecule binding specifically to a kidney cancer blood biomarker NNMT (Nicotinamide N-methyltransferase), (ii) a molecule binding specifically to a kidney cancer blood biomarker LCP1 (L-Plastin), and (iii) a molecule binding specifically to a kidney cancer blood biomarker NM23A (Non-metastatic cells 1 protein),
wherein in the *in vitro* diagnosis of kidney cancer a scoring method is used that uses blood concentrations of the kidney cancer blood biomarkers NNMT (Nicotinamide N-methyltransferase), LCP1 (L-Platin) and NM23A (Non-metastatic cells 1 protein), detected by the three specific binding molecules, and
wherein, according to the scoring method, individuals are assigned score 1 where the blood concentration of each kidney cancer blood biomarker exceeds a cut-off point (criterion), which is determined as a point where accuracy (AUC) of a receiver operating characteristic (ROC) curve of each biomarker is maximum, and score 0 where the blood concentration of each kidney cancer blood biomarker is equal to or less than the cut-off point (criterion), finally assigned a score ranging from 0 at minimum to 3 at maximum as the sum of 3 markers, with respect to a combination of the three biomarkers, wherein a cut-off value is given to score 1, and individuals are classified into kidney cancer patients where the score is higher than score 1 and into normal persons where the score is equal to or less than score 1.

10. The use of a kit according to claim 9, wherein the blood markers are detected from blood samples or processed blood samples.

11. The use of a kit according to claim 9, wherein the binding molecules specific to the kidney cancer blood biomarkers are antibodies.

12. The use of a kit according to claim 9, wherein the kit further comprises a support to which an antibody is immobilized or not.

13. The use of a kit according to claim 9, wherein the kit further comprises a secondary antibody or a detection antibody in a form conjugated with a label or enzyme allowing for signal measurement, or as an isolate.

14. The use of a kit according to claim 9, wherein the kit further comprises a wash buffer, a buffer for diluting the samples or the antibodies, an enzyme substrate, and a reaction stopper.

## Patentansprüche

1. Verwendung einer Kombination aus drei spezifischen Bindungsmolekülen von Nierenkrebsblutbiomarkern bei der in-vitro-Diagnose von Nierenkrebs, **dadurch gekennzeichnet, dass**
die drei spezifischen Bindungsmoleküle aus Antikörpern und Aptameren ausgewählt sind und aus: (i) einem Molekül, das spezifisch an einen Nierenkrebsblutbiomarker NNMT (Nicotinamid-N-Methyltransferase) bindet, (ii) einem Molekül, das spezifisch an einen Nierenkrebsblutbiomarker LCP1 (L-Plastin) bindet, und (iii) einem Molekül, das spezifisch an einen Nierenkrebsblutbiomarker NM23A (Non-Metastatic Cells 1 Protein) bindet, bestehen,
wobei in der in-vitro-Diagnose von Nierenkrebs ein Scoringverfahren verwendet wird, das die Blutkonzentrationen der Nierenkrebsblutbiomarker NNMT (Nicotinamid-N-Methyltransferase), LCP1 (L-Platin) und NM23A (Non-Metastatic Cells 1 Protein), detektiert durch die drei spezifischen Bindungsmoleküle, verwendet, und wobei, gemäß dem Scoringverfahren, Individuen die Punktzahl 1 zugewiesen wird, bei denen die Blutkonzentration jedes Nierenkrebsblutbiomarkers einen Cut-Off-Punkt (Kriterium) überschreitet, der als der Punkt bestimmt wird, an dem die Genauigkeit (AUC) einer Receiver-Operating-Characteristic(ROC)-Kurve von jedem Biomarker maximal ist, und Individuen die Punktzahl 0 zugewiesen wird, bei denen die Blutkonzentration jedes Nierenkrebsblutbiomarkers gleich oder kleiner als der Cut-Off-Punkt (Kriterium) ist, wobei Individuen, in Bezug auf eine Kombination der drei Biomarker, letztlich eine Punktzahl von 0 bis maximal 3 als Summe der 3 Marker zugewiesen wird, wobei der Punktzahl 1 ein Cut-off-Wert zugewiesen wird, und Individuen als Nierenkrebspatienten eingestuft werden, bei denen die Punktzahl größer als die Punktzahl 1 ist, und in normale Personen, bei denen die Punktzahl gleich oder kleiner als die Punktzahl 1 ist.

2. Verwendung der Kombination aus drei spezifischen Bindungsmolekülen von Nierenkrebsblutbiomarkern nach Anspruch 1, wobei (a) eine Blutprobe von einer Testperson oder eine verarbeitete Probe davon mit der Kombination aus drei spezifischen Bindungsmolekülen behandelt wird, um einzelne Komplexe zwischen den Nierenkrebsblutbiomarkern NNMT, LCP1 und NM23A und den jeweils dafür spezifischen Bindungsmolekülen zu bilden, und wobei (b) die Komplexe nachgewiesen werden.

3. Verwendung einer Kombination aus drei spezifischen Bindungsmolekülen von Nierenkrebsblutbiomarkern nach Anspruch 1 oder 2, wobei die verarbeitete Probe eine Plasma- oder Serumprobe ist.

4. Verwendung einer Kombination aus drei spezifischen Bindungsmolekülen von Nierenkrebsblutbiomarkern nach Anspruch 1 oder 2, wobei die Bindungsmoleküle Antikörper sind, die spezifisch für die Nierenkrebsblutbiomarker sind.

5. Verwendung einer Kombination aus drei spezifischen Bindungsmolekülen von Nierenkrebsblutbiomarkern nach Anspruch 4, wobei der Schritt (a) des Behandelns einer Blutprobe einer Testperson oder einer verarbeiteten Probe davon mit einer Kombination aus drei spezifischen Bindungsmolekülen umfasst:
(a-i) das Immobilisieren der Nierenkrebsblutbiomarkerspezifischen Antikörper als Einfangantikörper auf einem Träger; und
(a-ii) das Behandeln des Trägers mit dem immobilisierten Einfangantikörper mit einer Blutprobe oder einer verarbeiteten Probe davon, um die Bildung der Komplexe zwischen den Einfangantikörpern und den Nierenkrebsblutbiomarkern zu induzieren, und
wobei der Schritt (b) des Nachweisens der Komplexe umfasst:
(b-i) das Aufbringen eines Nachweisantikörpers, der mit einer Markierung oder einem Enzym konjugiert ist, das eine Signalmessung vermittelt oder bereitstellt, auf den Träger; und
(b-ii) das Messen des Signals, das durch die Markierung oder das Enzym vermittelt oder bereitgestellt wird.

6. Verwendung einer Kombination aus drei spezifischen Bindungsmolekülen von Nierenkrebsblutbiomarkern nach Anspruch 5, wobei der Träger eine Mikrokugel ist, wobei die Mikrokugel eine Harzperle ist.

7. Verwendung einer Kombination aus drei spezifischen Bindungsmolekülen von Nierenkrebsblutbiomarkern nach Anspruch 5, wobei die Markierung, die eine Signalmessung vermittelt oder bereitstellt, Biotin ist.

8. Verwendung einer Kombination aus drei spezifischen Bindungsmolekülen von Nierenkrebsblutbiomarkern nach Anspruch 4, wobei der Schritt (a) des Behandelns einer Blutprobe einer Testperson oder einer verarbeiteten Probe davon mit einer Kombination aus drei spezifischen Bindungsmolekülen umfasst:
(a-i) das Immobilisieren der Blutprobe oder der verarbeiteten Probe auf einem Träger; und
(a-ii) das Behandeln des Trägers mit der immobilisierten Probe mit den primären Antikörpern gegen die Nierenkrebsblutbiomarker; und
wobei der Schritt (b) des Nachweisens des Komplexes umfasst:
(b-i) das Aufbringen eines enzymkonjugierten sekundären Antikörpers auf den Träger; und
(b-ii) Messen des Enzyms auf Aktivität.

9. Verwendung eines Kits, umfassend eine Kombination aus drei spezifischen Bindungsmolekülen bei der in-vitro-Diagnose von Nierenkrebs, **dadurch gekennzeichnet, dass** die drei spezifischen Bindungsmoleküle aus Antikörpern und Aptameren ausgewählt sind und aus: (i) einem Molekül, das spezifisch an einen Nierenkrebsblutbiomarker NNMT (Nicotinamid-N-Methyltransferase) bindet, (ii) einem Molekül, das spezifisch an einen Nierenkrebsblutbiomarker LCP1 (L-Plastin) bindet, und (iii) einem Molekül, das spezifisch an einen Nierenkrebsblutbiomarker NM23A (Non-Metastatic Cells 1 Protein) bindet, bestehen,
wobei in der in-vitro-Diagnose von Nierenkrebs ein Scoringverfahren verwendet wird, das die Blutkonzentrationen der Nierenkrebsblutbiomarker NNMT (Nicotinamid-N-Methyltransferase), LCP1 (L-Platin) und NM23A (Non-Metastatic Cells 1 Protein), detektiert durch die drei spezifischen Bindungsmoleküle, verwendet, und wobei, gemäß dem Scoringverfahren, Individuen die Punktzahl 1 zugewiesen wird, bei denen die Blutkonzentration jedes Nierenkrebsblutbiomarkers einen Cut-Off-Punkt (Kriterium) überschreitet, der als der Punkt bestimmt wird, an dem die Genauigkeit (AUC) einer Receiver-Operating-Characteristic(ROC)-Kurve von jedem Biomarker maximal ist, und Individuen die Punktzahl 0 zugewiesen wird, bei denen die Blutkonzentration jedes Nierenkrebsblutbiomarkers gleich oder kleiner als der Cut-Off-Punkt (Kriterium) ist, wobei Individuen, in Bezug auf eine Kombination der drei Biomarker, letztlich eine Punktzahl von 0 bis maximal 3 als Summe der 3 Marker zugewiesen wird, wobei der Punktzahl 1 ein Cut-off-Wert zugewiesen wird, und Individuen als Nierenkrebspatienten eingestuft werden, bei denen die Punktzahl größer als die Punktzahl 1 ist, und in normale Personen, bei denen die Punktzahl gleich oder kleiner als die Punktzahl 1 ist.

10. Verwendung eines Kits nach Anspruch 9, wobei die Blutmarker aus Blutproben oder verarbeiteten Blutproben nachgewiesen werden.

11. Verwendung eines Kits nach Anspruch 9, wobei die für die Nierenkrebsblutbiomarker spezifischen Bindungsmoleküle Antikörper sind.

12. Verwendung eines Kits nach Anspruch 9, wobei das Kit ferner einen Träger umfasst, an den ein Antikörper immobilisiert ist oder nicht.

13. Verwendung eines Kits nach Anspruch 9, wobei das Kit ferner einen sekundären Antikörper oder einen Nachweisantikörper in einer Form, die mit einer Markierung oder einem Enzym konjugiert ist, die eine Signalmessung ermöglichen, oder als Isolat umfasst.

14. Verwendung eines Kits nach Anspruch 9, wobei das Kit ferner einen Waschpuffer, einen Puffer zum Verdünnen der Proben oder der Antikörper, ein Enzymsubstrat und einen Reaktionsstopper umfasst.

## Revendications

1. Utilisation d'une combinaison de trois molécules liantes spécifiques des biomarqueurs sanguins de cancer du rein dans le diagnostic *in vitro* d'un cancer du rein, **caractérisée en ce que** :
les trois molécules liantes spécifiques sont choisies parmi les anticorps et les aptamères et consistent en : (i) une molécule se liant spécifiquement à un biomarqueur sanguin de cancer du rein NNMT (Nicotinamide N-méthyltransférase), (ii) une molécule se liant spécifiquement à un biomarqueur sanguin de cancer du rein LCP1 (L-Plastin), et (iii) une molécule se liant spécifiquement à un biomarqueur sanguin de cancer du rein NM23A (protéine de cellules non métastatiques 1),
dans laquelle, dans le diagnostic *in vitro* d'un cancer du rein, l'on utilise un procédé de notation qui utilise les concentrations sanguines des biomarqueurs sanguins de cancer du rein NNMT (Nicotinamide N-méthyltransférase), LCP1 (L-Plastin) et NM23A (protéine de cellules non métastatiques 1), détectés par les trois molécules de liaison spécifiques, et
dans laquelle, d'après le procédé de notation, les personnes reçoivent la note 1 lorsque la concentration sanguine de chaque biomarqueur sanguin de cancer du rein dépasse un point seuil (critère), qui est déterminé comme étant un point où la précision (AUC) d'une courbe de la fonction d'efficacité du récepteur (ROC) de chaque biomarqueur est maximale, et la note 0 lorsque la concentration sanguine de chaque biomarqueur sanguin de cancer du rein est égale ou inférieure au point seuil (critère), reçoivent enfin une note allant de 0 au minimum à 3 au maximum en tant que somme des 3 marqueurs, pour ce qui est d'une combinaison des trois biomarqueurs, dans lequel une valeur seuil est donnée à la note 1, et les personnes sont classifiées en patients avec un cancer du rein lorsque la note est supérieure à la note 1 et en personnes normales lorsque la note est égale ou inférieure à la note 1.

2. Utilisation d'une combinaison de trois molécules de liaison spécifiques de biomarqueurs sanguins de cancer du rein selon la revendication 1, dans laquelle (a) un échantillon de sang d'un sujet ou un échantillon transformé de celui-ci est traité avec la combinaison de trois molécules de liaison spécifiques pour former des complexes individuels entre les biomarqueurs sanguins de cancer du rein NNMT, LCP1 et NM23A et les molécules de liaison respectivement spécifiques de ceux-ci, et dans laquelle (b) les complexes sont détectés.

3. Utilisation d'une combinaison de trois molécules de liaison spécifiques de biomarqueurs sanguins de cancer du rein selon la revendication 1 ou 2, dans laquelle l'échantillon transformé est un échantillon de plasma ou de sérum.

4. Utilisation d'une combinaison de trois molécules de liaison spécifiques de biomarqueurs sanguins de cancer du rein selon la revendication 1 ou 2, dans laquelle les molécules de liaison sont des anticorps spécifiques des biomarqueurs sanguins de cancer du rein, respectivement.

5. Utilisation d'une combinaison de trois molécules de liaison spécifiques de biomarqueurs sanguins de cancer du rein selon la revendication 4, dans laquelle l'étape (a) consistant à traiter un échantillon de sang d'un sujet ou un échantillon transformé de celui-ci avec un combinaison de trois molécules de liaison spécifiques comprend :
(a-i) l'immobilisation des anticorps spécifiques des biomarqueurs sanguins de cancer du rein en tant qu'anticorps de capture sur un support ; et
(a-ii) le traitement du support à anticorps de capture immobilisés avec un échantillon de sang ou un échantillon de sang transformé de celui-ci pour provoquer la formation des complexes entre les anticorps de capture et les biomarqueurs sanguins de cancer du rein,
et
dans laquelle l'étape (b) consistant à détecter les complexes comprend :
(b-i) l'application sur le support d'un anticorps de détection conjugué à un marqueur ou une enzyme qui médie ou fournit une mesure de signal ; et
(b-ii) la mesure du signal médié ou fourni par le marqueur ou l'enzyme.

6. Utilisation d'une combinaison de trois molécules de liaison spécifiques de biomarqueurs sanguins de cancer du rein selon la revendication 5, dans laquelle le support est une microsphère, la microsphère étant une perle de résine.

7. Utilisation d'une combinaison de trois molécules de liaison spécifiques de biomarqueurs sanguins de cancer du rein selon la revendication 5, dans laquelle le marqueur qui médie ou fournit une mesure de signal est la biotine.

8. Utilisation d'une combinaison de trois molécules de liaison spécifiques de biomarqueurs sanguins de cancer du rein selon la revendication 4, dans laquelle l'étape (a) consistant à traiter un échantillon de sang d'un sujet ou un échantillon transformé de celui-ci avec une combinaison de trois molécules de liaison spécifiques comprend :
(a-i) l'immobilisation de l'échantillon de sang ou de l'échantillon transformé sur un support ; et
(a-ii) le traitement du support à échantillon immobilisé avec les anticorps primaires dirigés contre les biomarqueurs sanguins de cancer du rein ; et
dans laquelle l'étape (b) consistant à détecter le complexe comprend :
(b-i) l'application sur le support d'un anticorps secondaire conjugué à une enzyme ;
et
(b-ii) la mesure de l'enzyme quant à l'activité.

9. Utilisation d'un kit comprenant une combinaison de trois molécules de liaison spécifiques dans le diagnostic *in vitro* d'un cancer du rein, **caractérisée en ce que** les trois molécules de liaison spécifiques sont choisies parmi les anticorps et les aptamères et consistent en : (i) une molécule se liant spécifiquement à un biomarqueur sanguin de cancer du rein NNMT (Nicotinamide N-méthyltransférase), (ii) une molécule se liant spécifiquement à un biomarqueur sanguin de cancer du rein LCP1 (L-Plastin), et (iii) une molécule se liant spécifiquement à un biomarqueur sanguin de cancer du rein NM23A (protéine de cellules non métastatiques 1),
dans laquelle, dans le diagnostic *in vitro* d'un cancer du rein, l'on utilise un procédé de notation qui utilise les concentrations sanguines des biomarqueurs sanguins de cancer du rein NNMT (Nicotinamide N-méthyltransférase), LCP1 (L-Plastin) et NM23A (protéine de cellules non métastatiques 1), détectés par les trois molécules de liaison spécifiques, et
dans laquelle, d'après le procédé de notation, les personnes reçoivent la note 1 lorsque la concentration sanguine de chaque biomarqueur sanguin de cancer du rein dépasse un point seuil (critère), qui est déterminé comme étant un point où la précision (AUC) d'une courbe de la fonction d'efficacité du récepteur (ROC) de chaque biomarqueur est maximale, et la note 0 lorsque la concentration sanguine de chaque biomarqueur sanguin de cancer du rein est égale ou inférieure au point seuil (critère), reçoivent enfin une note allant de 0 au minimum à 3 au maximum en tant que somme des 3 marqueurs, pour ce qui est d'une combinaison des trois biomarqueurs, dans lequel une valeur seuil est donnée à la note 1, et les personnes sont classifiées en patients avec un cancer du rein lorsque la note est supérieure à la note 1 et en personnes normales lorsque la note est égale ou inférieure à la note 1.

10. Utilisation d'un kit selon la revendication 9, dans laquelle les marqueurs sanguins sont détectés à partir d'échantillons de sang ou d'échantillons de sang transformé.

11. Utilisation d'un kit selon la revendication 9, dans laquelle les molécules de liaison spécifiques aux biomarqueurs sanguins de cancer du rein sont des anticorps.

12. Utilisation d'un kit selon la revendication 9, dans laquelle le kit comprend en outre un support sur lequel un anticorps est immobilisé ou pas.

13. Utilisation d'un kit selon la revendication 9, dans laquelle le kit comprend en outre un anticorps secondaire ou un anticorps de détection sous une forme conjuguée à un marqueur ou une enzyme permettant la mesure d'un signal, ou sous la forme d'un isolat.

14. Utilisation d'un kit selon la revendication 9, dans laquelle le kit comprend en outre un tampon de lavage, un tampon pour diluer les échantillons ou les anticorps, un substrat enzymatique et un agent mettant fin à la réaction.
